# EUROPÄISCHE PATENTSCHRIFT

(11) **EP 0 716 070 B1**
(45) Veröffentlichungstag und Bekanntmachung des Hinweises auf die Patenterteilung: **12.08.1998**
(21) Anmeldenummer: 95118743.4
(22) Anmeldetag: 29.11.1995
(51) Int. Cl.: C07C 45/41, C07C 47/02, C07C 47/32

(54) **Verfahren zur Herstellung von Aldehyden**
Process for the preparation of aldehydes
Procédé pour la préparation d'aldéhydes

(30) Priorität: 08.12.1994 DE 4443704
(43) Veröffentlichungstag der Anmeldung: 12.06.1996
(73) Patentinhaber: BASF AKTIENGESELLSCHAFT, 67056 Ludwigshafen (DE)
(72) Erfinder: Schnurr, Werner, Dr., D-67273 Herxheim (DE); Fischer, Rolf, Dr., D-69121 Heidelberg (DE); Wulff-Döring, Joachim, Dr., D-67227 Frankenthal (DE); Hesse, Michael, Dr., D-67105 Schifferstadt (DE)

(56) Entgegenhaltungen:
- EP-A- 0 150 961
- US-A- 4 328 373

## Beschreibung

Die vorliegende Erfindung betrifft ein Verfahren zur Herstellung von Aldehyden durch Umsetzung entsprechender Carbonsäuren oder deren Ester mit Wasserstoff in der Gasphase in Gegenwart von Zirkondioxid und Elemente der Lanthaniden enthaltenden Katalysatoren.

Es ist bekannt, Carbonsäuren wie Benzoesäure oder Cyclohexancarbonsäure oder ihre Ester durch Hydrierung in der Gasphase in die entsprechenden Aldehyde zu überführen.

Aus der US-A-3,935,265 ist bekannt, daß man Alkylester aromatischer Carbonsäuren bei 400 bis 600°C an Al₂O₃ mit Wasserstoff hydrieren kann. Beispielsweise wird Benzoesäuremethylester mit einer Selektivität von 37 % (Umsatz: 39 %) zu Benzaldehyd umgesetzt. Weiterhin werden z.B. Ru/Sn- (EP-A-539 274), Manganoxid-(EP-A-290 096, US-A-4,585,899), Eisenoxid- (EP-A-304 853), Vanadiumoxid und/oder Titandioxid- (US-A-4,950,799, EP-A-414065), Cu/Y₂O₃- (US-A-4,585,900), Cr₂O₃/ZrO₂- (EP-A-150961, EP 439115), Cr₂O₃- (US 5306845) oder Lanthanidoxide/Al₂O₃-Katalysatoren (US-A-4,328,373, EP-A-101 111) für die Hydrierung von aromatischen und aliphatischen Carbonsäuren eingesetzt.

Bei den bekannten Hydrierverfahren werden in den meisten Fällen, zum Teil bedingt durch sehr hohe Hydriertemperaturen, nur unbefriedigende Ausbeuten und Selektivitäten erzielt.

Der vorliegenden Erfindung lag daher die Aufgabe zugrunde, den zuvor genannten Nachteilen abzuhelfen.

Demgemäß wurde ein neues und verbessertes Verfahren zur Herstellung von Aldehyden der allgemeinen Formel I 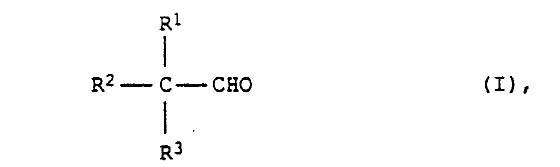 in der
- R¹,R² und R³: Wasserstoff, C₁- bis C₆-Alkyl, C₃- bis C₈-Cycloalkyl, Aryl, C₇- bis C₁₂-Alkylphenyl, C₇- bis C₁₂-Phenylalkyl und R¹ und R² zu einem 3- bis 7-gliedrigen cycloaliphatischen Ring verknüpft sind und
- R¹ und R³: C₁- bis C₄-Alkoxy, Phenoxy, Methylamino, Dimethylamino und Halogen und
- R¹: zusätzlich Hydroxy und Amino
bedeuten, gefunden, welches dadurch gekennzeichnet ist, daß man Carbonsäuren oder deren Ester der allgemeinen Formel II 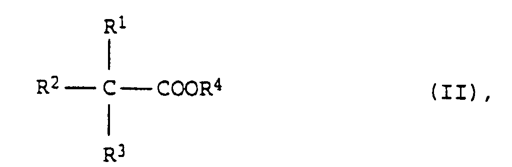 in der
- R¹,R² und R³: die obengenannte Bedeutung haben und
- R⁴: Wasserstoff oder C₁- bis C₆-Alkyl, C₃- bis C₈-Cycloalkyl, Aryl, C₇- bis C₁₂-Alkylphenyl, C₇- bis C₁₂-Phenylalkyl,
bedeutet, mit Wasserstoff in der Gasphase bei Temperaturen von 200 bis 450°C und Drücken von 0,1 bis 20 bar in Gegenwart eines Katalysators, dessen katalytisch aktive Masse 80 bis 99,9 Gew.-% zirkonoxid und 0,1 bis 20 Gew.-% eines oder mehrerer Elemente der Lanthaniden enthält, umsetzt.

Das erfindungsgemäße Verfahren läßt sich wie folgt durchführen:

Die erfindungsgemäße Hydrierung der Carbonsäuren oder deren Ester II mit Wasserstoff in Gegenwart eines Katalysators, dessen katalytisch aktive Masse 60 bis 99,9, insbesondere 80 bis 99,9 Gew.-% zirkonoxid und 0,1 bis 40, insbesondere 0,1 bis 20 Gew.-% eines oder mehrerer Elemente der Lanthaniden enthält, wird in der Regel bei Temperaturen von 200 bis 450°C, bevorzugt 250 bis 400°C, besonders bevorzugt 300 bis 380°C und Drücken von 0,1 bis 20 bar, bevorzugt 0,7 bis 5 bar, besonders bevorzugt Atmosphärendruck (Normaldruck) durchgeführt. Die erforderliche Temperatur und der erforderliche Druck sind abhängig von der Katalysatoraktivität und der thermischen Stabilitat von Edukt und Produkt.

Als Katalysatoren eignen sich Trägerkatalysatoren, bevorzugt Vollkatalysatoren von Zirkondioxid in kubischer, tetragonaler oder monokliner Phase, bevorzugt in monokliner Phase, die bevorzugt mit einem oder mehreren Elementen aus der Lanthaniden-Reihe dotiert sind. Die katalytisch aktive Masse enthält in der Regel 80 bis 99,9 Gew.-%, bevorzugt 90 bis 99,9 Gew.-%, besonders bevorzugt 92 bis 99 Gew.-% Zirkonoxid und 0,1 bis 20 Gew.-% eines oder mehrerer Elemente der Lanthaniden, bevorzugt 0,1 bis 10Gew.-% Lanthan, Zer, Praseodym, Neodym, Samarium, Europium oder deren Gemische, besonders bevorzugt 1 bis 8 Gew.-% Lanthan-(III)-oxid. Die Dotierung erfolgt in der Regel durch Tränken des Zirkondioxids mit Salzlösungen (wäßrig oder alkoholisch) der Lanthaniden.

Der Katalysator kann zusätzlich weitere Dotierungen (z.B. Chrom, Eisen, Yttrium, Mangan) in Mengen von 0,001 bis 10 Gew.-% enthalten. Bevorzugt sind Katalysatoren ohne solche Zusätze.

Die BET-Oberfläche des Zirkonoxids kann in weiten Grenzen schwanken, beträgt in der Regel 5 bis 150 m²/g, bevorzugt 20 bis 150m²/g, besonders bevorzugt 40 bis 120 m²/g.

Derartige Katalysatoren werden in bekannter Weise z. B. durch Tränken vorgeformter Träger wie Pellets, Kugeln oder Stränge, Trocknen und Calcinieren hergestellt.

Die bevorzugt verwendeten Trägerkatalysatoren zeigen über einen längeren Zeitraum hohe Aktivität. Desaktivierte Katalysatoren lassen sich durch Behandlung mit molekularen Sauerstoff enthaltenden Gasen, z.B. Luft, bei Temperaturen von 350 bis 500°C regenerieren.

Im allgemeinen hält man eine Katalysatorbelastung von 0,01 bis 10, vorzugsweise 0,01 bis 3 kg Carbonsäure(ester) je kg Katalysator und Stunde ein.

Die Wasserstoffkonzentration im Eingangsgas richtet sich nach der Carbonsäure(ester)konzentration. Das Molverhältnis von Wasserstoff zu Carbonsäure(ester) beträgt in der Regel 2:1 bis 100:1, bevorzugt 10:1 bis 70:1. Als Wasserstoffquelle kann auch Ameisensäure eingesetzt werden.

Vorteilhaft kann auch der Zusatz eines inerten Verdünnungsmittels sein. In der Regel werden Stickstoff, Wasser oder gasförmige, unter den Reaktionsbedingungen inerte Verbindungen wie z.B. Kohlenwasserstoffe, Aromaten oder Ether verwendet.

Die Umsetzung kann in der Gasphase, kontinuierlich als Festbettreaktion mit fest angeordnetem Katalysator, beispielsweise in Sumpf- oder Rieselfahrweise oder als Wirbelbettreaktion mit in auf- und abwirbelnder Bewegung befindlichem Katalysator durchgeführt werden. Bevorzugt ist das Arbeiten im Festbett.

Zur Steigerung der Selektivität können bei der Hydrierung gebildete Nebenprodukte, z.B. Alkohole, in die Synthese zurückgeführt werden.

Die Substituenten R¹, R², R³ und R⁴ in den Verbindungen I und II haben unabhängig voneinander die folgenden Bedeutungen:
R¹, R², R³ und R⁴
- Wasserstoff,
- C₁- bis C₆-Alkyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3, 4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl, bevorzugt 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl und 3,5-Dimethylphenyl, besonders bevorzugt 2-Methylphenyl, 3-Methylphenyl, 4-Methylphenyl,
- C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, bevorzugt Benzyl, 1-Phenethyl und 2-Phenethyl, besonders bevorzugt Benzyl,
R¹ und R³
- C₁- bis C₄-Alkoxy wie Methoxy, Ethoxy, n-Propoxy, iso-Propoxy, n-Butoxy, iso-Butoxy, sec.-Butoxy und tert.-Butoxy, bevorzugt Methoxy, Ethoxy, n-Propoxy und iso-Propoxy, besonders bevorzugt Methoxy und Ethoxy,
- Phenoxy,
- Methylamino,
- Dimethylamino,
- Halogen wie Fluor, Chlor, Brom und Jod, bevorzugt Fluor, Chlor und Brom, besonders bevorzugt Chlor und Brom und
R¹
- Hydroxy,
- Amino,
R¹ und R² zusammen einen 3- bis 7-gliedrigen cycloaliphatischen Ring wie Cyclopropyl, Cyclobutyl, Cyclopentyl, Cyclohexyl und Cycloheptyl, bevorzugt Cyclopentyl, Cyclohexyl und Cycloheptyl, besonders bevorzugt Cyclopentyl und Cyclohexyl, wobei bevorzugt R³ Wasserstoff oder C₁- bis C₆-Alkyl ist.

Als Einsatzstoffe dienen Carbonsäuren bzw. Carbonsäureester der Formel II, z.B. Phenylessigsäure, Diphenylessigsäure, Triphenylessigsäure, Phenylessigsäuremethylester, Pivalinsäure, iso-Buttersäure, Phenylmethylessigsäure, Cyclohexancarbonsäure-(ester), Cyclopentancarbonsäure(ester), Dimethylhydroxyessig-säure, Diphenylchloressigsäure, Dimethylmethoxyessigsäure.

Mit Hilfe des erfindungsgemäßen Verfahrens ist es möglich, bisher schwer zugängliche Aldehyde auf einfache Weise selektiv herzustellen.

Die Aldehyde I eignen sich als Geruchs- und Geschmacksstoffe bzw. als Zwischenprodukte z.B. für Pharma- und Pflanzenschutzwirkstoffe (Ullmann's Encyclpedia of Industrial Chemistry, Vol.A3, Seite 469 bis 474).

### Beispiele

### Katalysatorherstellung

### Beispiel 1

Monoklines Zirkondioxid (BET-Oberfläche: 53 m²/g) in Form von Tabletten (Katalysator A) wurde mit einer wäßrigen Lösung Lanthannitrat unter guter Durchmischung getränkt und 2 h bei Raumtemperatur gehalten. Anschließend wurde der Katalysator 15 Stunden bei 120°C getrocknet und anschließend 2 bis 4 Stunden bei 400 bis 500°C getempert.

Der so hergestellten Katalysator hatte einen Lanthan-Gehalt von 3 Gew.-%.

### Beispiel 2

Pro Stunde wurden 11 g Pivalinsäure (als Schmelze) mit 1001/h Wasserstoff verdampft und bei 330°C über 100 ml (128 g) Katalysator A in Rieselfahrweise geleitet. Der gasförmige Reaktionsaustrag wurde in Kühlfallen kondensiert und gaschromatographisch analysiert. Die Pivalaldehyd-Ausbeute betrug 98% (Umsatz 100 %). Als Nebenprodukt entstand 1 % des entsprechenden Alkohols (rückführbar).

### Beispiel 3

Analog Beispiel 2 wurden die in der nachfolgenden Tabelle aufgeführten Carbonsäuren bzw. Carbonsäureester umgesetzt und die Ergebnisse der Umsetzung zusammengestellt.

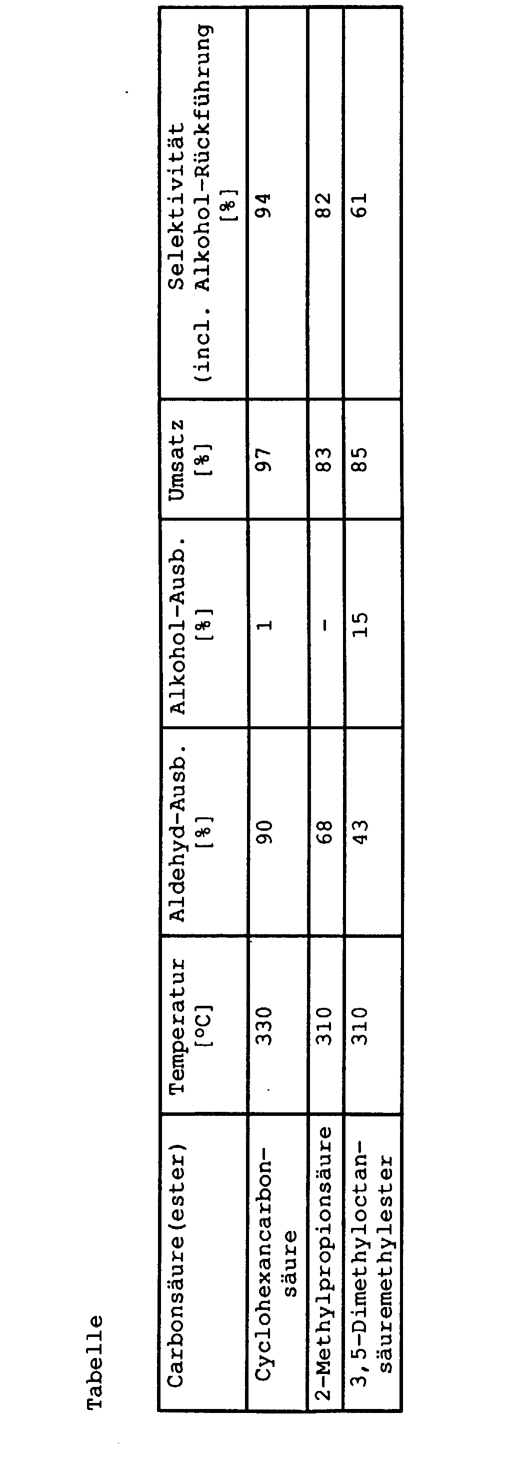

## Patentansprüche

1. Verfahren zur Herstellung von Aldehyden der allgemeinen Formel I 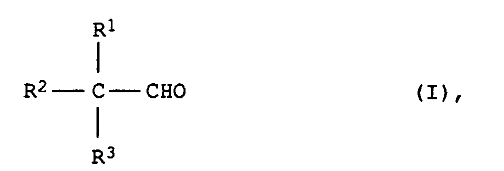 in der
R¹,R² und R³ Wasserstoff, C₁- bis C₆-Alkyl, C₃- bis C₈-Cycloalkyl, Aryl, C₇- bis C₁₂-Alkylphenyl, C₇- bis C₁₂-Phenylalkyl und R¹ und R² zu einem 3- bis 7-gliedrigen cycloaliphatischen Ring verknüpft sind,
R¹ und R³ C₁- bis C₄-Alkoxy, Phenoxy, Methylamino, Dimethylamino und Halogen und
R¹ zusätzlich Hydroxy und Amino
bedeuten, dadurch gekennzeichnet, daß man Carbonsäuren oder deren Ester der allgemeinen Formel II 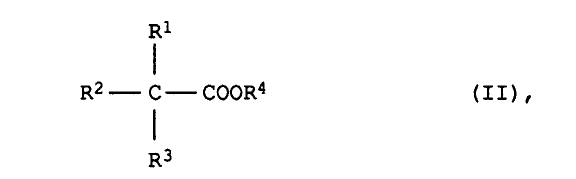 in der
R¹,R² und R³ die obengenannte Bedeutung haben und
R⁴ Wasserstoff, C₁- bis C₆-Alkyl, C₃- bis C₈-Cycloalkyl, Aryl, C₇- bis C₁₂-Alkylphenyl, oder C₇- bis C₁₂-Phenylalkyl,
bedeutet, mit Wasserstoff in der Gasphase bei Temperaturen von 200 bis 450°C und Drücken von 0,1 bis 20 bar in Gegenwart eines Katalysators, dessen katalytisch aktive Masse 80 bis 99,9 Gew.-% Zirkondioxid und 0,1 bis 20 Gew.-% eines oder mehrere Elemente der Lanthaniden enthält, umsetzt.

2. Verfahren zur Herstellung von Aldehyden nach Anspruch 1, dadurch gekennzeichnet, daß der Katalysator als katalytisch aktive Masse 90 bis 99,9 Gew.-% Zirkondioxid und als Element der Lanthaniden 0,1 bis 10 Gew.-% Lanthan, Zer, Praseodym, Neodym, Samarium, Europium oder deren Gemische enthält.

3. Verfahren zur Herstellung von Aldehyden nach Anspruch 1, dadurch gekennzeichnet, daß der Katalysator als katalytisch aktive Masse 92 bis 99 Gew.-% Zirkondioxid und 1 bis 8 Gew.-% Lanthan-(III)-oxid enthält.

4. Verfahren zur Herstellung von Aldehyden nach Anspruch 1, dadurch gekennzeichnet, daß das Zirkondioxid monoklin ist.

5. Verfahren zur Herstellung von Aldehyden nach Anspruch 1, dadurch gekennzeichnet, daß man Pivalinsäure, iso-Buttersäure, Cyclohexancarbonsäure oder ihre Ester zum entsprechenden Aldehyd hydriert.

6. Verfahren zur Herstellung von Aldehyden nach Anspruch 1, dadurch gekennzeichnet, daß das Molverhältnis von Wasserstoff zu Carbonsäure(ester) 2:1 bis 100:1 beträgt.

7. Verfahren zur Herstellung von Aldehyden nach Anspruch 1, dadurch gekennzeichnet, daß man die Umsetzung im Festbett durchführt.

## Claims

1. A process for preparing aldehydes of the general formula I 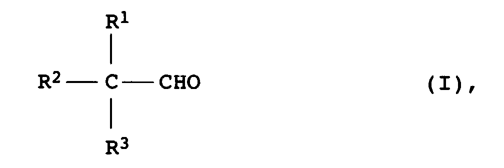 where
R¹, R² and R³ are each hydrogen, C₁-C₆-alkyl, C₃-C₈-cycloalkyl, aryl, C₇-C₁₂-alkylphenyl, C₇-C₁₂-phenylalkyl and R¹ and R² are joined together to form a 3-, 4-, 5-, 6- or 7-membered cycloaliphatic ring,
R¹ and R³ are each C₁-C₄-alkoxy, phenoxy, methylamino, dimethylamino or halogen, and
R¹ is additionally hydroxyl or amino,
which comprises reacting a carboxylic acid or ester of the general formula II 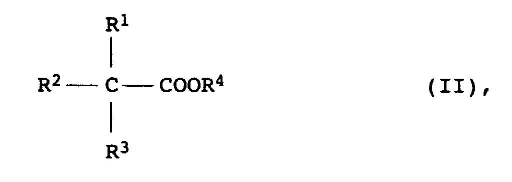 where
R¹, R² and R³ are each as defined above, and
R⁴ is hydrogen, C₁-C₆-alkyl, C₃-C₈-cycloalkyl, aryl, C₇-C₁₂-alkylphenyl or C₇-C₁₂-phenylalkyl,
with hydrogen in the gas phase at temperatures from 200 to 450°C and pressures from 0.1 to 20 bar in the presence of a catalyst whose catalytically active mass comprises from 80 to 99.9% by weight of zirconium dioxide and from 0.1 to 20% by weight of one or more elements of the lanthanides.

2. A process as claimed in claim 1, wherein the catalytically active mass of the catalyst comprises from 90 to 99.9% by weight of zirconium dioxide and from 0.1 to 10% by weight of lanthanum, cerium, praseodymium, neodymium, samarium, europium or mixtures thereof.

3. A process as claimed in claim 1, wherein the catalytically active mass of the catalyst comprises from 92 to 99% by weight of zirconium dioxide and from 1 to 8% by weight of lanthanum(III) oxide.

4. A process as claimed in claim 1, wherein the zirconium dioxide is monoclinic.

5. A process as claimed in claim 1, wherein the carboxylic acid or ester used is pivalic acid or ester, isobutyric acid or ester, or cyclohexanecarboxylic acid or ester.

6. A process as claimed in claim 1, wherein the molar ratio of hydrogen to carboxylic acid or ester is within the range from 2:1 to 100:1.

7. A process as claimed in claim 1, wherein the reaction is carried out in a fixed bed.

## Revendications

1. Procédé pour la préparation d'aldéhydes de formule générale I 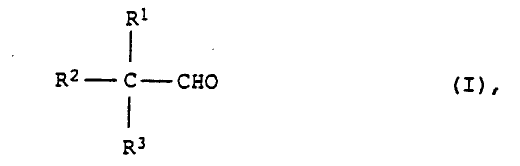 dans laquelle
R¹, R² et R³ représentent l'hydrogène ou un groupe alkyle en C₁-C₆, cycloalkyle en C₃-C₈, aryle, alkylphényle en C₇-C₁₂, phénylalkyle en C₇-C₁₂ et R¹ et R² sont réunis dans un noyau cycloaliphatique ayant 3 à 7 membres,
R¹ et R³ désignent un groupe alcoxy en C₁-C₄, phénoxy, méthylamino, diméthylamino et halogène et
R¹ désigne en outre un groupe hydroxy et amino,
caractérisé par le fait qu'on fait réagir des acides carboxyliques ou leurs esters de formule générale II 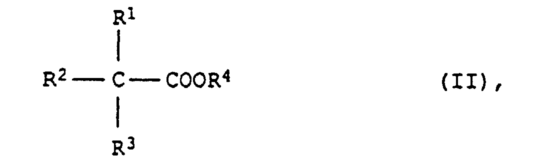 dans laquelle
R¹, R² et R³ ont la signification indiquée plus haut et
R⁴ représente l'hydrogène ou un groupe alkyle en C₁-C₆, cycloalkyle en C₃-C₈, aryle, alkylphényle en C₇-C₁₂ ou phénylalkyle en C₇-C₁₂,
avec de l'hydrogène en phase gazeuse, à des températures de 200 à 450°C et sous des pressions de 0,1 à 20 bar, en présence d'un catalyseur dont la masse catalytiquement active contient 80 à 99,9 % en poids de dioxyde de zirconium et 0,1 à 20 % en poids d'un ou plusieurs éléments des lanthanides.

2. Procédé pour la préparation d'aldéhydes selon la revendication 1, caractérisé par le fait que le catalyseur contient comme masse catalytiquement active 90 à 99,9 % en poids de dioxyde de zirconium et comme élément des lanthanides 0,1 à 10 % en poids de lanthane, de cérium, de praséodyme, de néodyme, de samarium, d'europium ou de leurs mélanges.

3. Procédé pour la préparation d'aldéhydes selon la revendication 1, caractérisé par le fait que le catalyseur contient comme masse catalytiquement active 92 à 99 % en poids de dioxyde de zirconium et 1 à 8 % en poids d'oxyde de lanthane(III).

4. Procédé pour la préparation d'aldéhydes selon la revendication 1, caractérisé par le fait que le dioxyde de zirconium est monoclinique.

5. Procédé pour la préparation d'aldéhydes selon la revendication 1, caractérisé par le fait qu'on hydrogène de l'acide pivalique, de l'acide isobutyrique, de l'acide cyclohexanecarboxylique ou leurs esters en l'aldéhyde correspondant.

6. Procédé pour la préparation d'aldéhydes selon la revendication 1, caractérisé par le fait que le rapport molaire de l'hydrogène à l'acide(ester) carboxylique est de 2:1 à 100:1.

7. Procédé pour la préparation d'aldéhydes selon la revendication 1, caractérisé par le fait qu'on effectue la réaction en lit fixe.
